# EUROPEAN PATENT APPLICATION

(11) **EP 4 116 705 A1**
(43) Date of publication of application: **11.01.2023**
(21) Application number: 22181860.2
(22) Date of filing: 29.06.2022
(51) Int. Cl.: G01N 27/327

(54) **BIOSENSOR**

(30) Priority: 05.07.2021 JP 2021111534; 22.06.2022 JP 2022100264
(71) Applicant: ARKRAY, Inc., Kyoto-shi, Kyoto 601-8045 (JP)
(72) Inventor: Masuda, Katsuhisa, Kyoto-shi, Kyoto, 602-0008 (JP)
(74) Representative: Hoffmann Eitle

(57) **Abstract**

A biosensor for measuring a measuring object component contained in a sample includes a substrate having an upper surface on which a measurement region is formed, a spacer which has a cutout portion obtained by cutting out a part of the spacer and which is stacked on the upper surface so that the measurement region is located inside the cutout portion, and a cover which has an air hole obtained by cutting out a part of the cover and which is stacked on the spacer while covering the cutout portion. Further, the substrate, spacer, and cover form a flow path for the sample, the flow path being formed to have a supply port for supplying the sample and communicate with the air hole. The side wall of the flow path is constituted by the cutout portion, and a downstream edge portion of the cutout portion forming a downstream end of the flow path and an exposed portion, which is a part of the spacer continuous from the downstream edge portion, protrude into the air hole in a top view of the air hole.

## Description

### BACKGROUND

### 1. Technical Field

The present invention relates to a biosensor.

### 2. Description of the Related Art

A biosensor is provided with a flow path for supplying a sample supplied from a supply port to a measurement region, and an air hole for discharging the air that is filled in the flow path and pushed out by the sample. In a conventional biosensor, the air hole is formed to be larger on the surface facing the flow path than on the surface opposite thereto (see, for example, Japanese Patent Application Publication No. 2005-249491).

Further, a biosensor is known in which the height of the edge of the air hole facing the flow path of the sample is lower than that on the surface opposite thereto (see, for example, Japanese Translation of PCT Application No. 2005-274252). Furthermore, there is a biosensor in which the upper surface of the flow path around the air hole is water repellent and the downstream end of the flow path communicates with the air hole (see, for example, Japanese Patent Application Publication No. 2013-257310). In addition, a sensor strip is known in which a plurality of air holes is provided in an intermediate point of the flow path (see, for example, Japanese Patent No. 4555686).

### SUMMARY

Since one side of the biosensor has a size of several millimeters to several tens of millimeters, and one side of the air hole (diameter in the case of a circle) is several tens of microns to several thousand microns, there is a limit to the processing accuracy of the air hole for the flow path of a sample. Therefore, it is necessary to consider that the air hole may be misaligned when the biosensor is manufactured. In the related art shown in Japanese Patent Application Publication No. 2005-249491 and Japanese Translation of PCT Application No. 2005-274252, the air hole is formed at a position slightly upstream from the downstream end of the flow path, and the influence of the air hole misalignment is small. However, this shape has a problem that bubbles tend to remain in the flow path downstream of the air hole. In the related art shown in Japanese Patent Application Publication No. 2013-257310, the biosensor is formed so that the downstream end of the flow path coincides with the downstream end of the air hole. This shape has a problem that bubbles tend to remain downstream of the air hole when the air hole is formed so as to be displaced upstream of the flow path. In addition, bubbles tend to remain in the downstream end portion where the air hole is not formed. Further, in the related art shown in Japanese Patent No. 4555686, although the influence of the air hole misalignment is small, since a plurality of air holes is provided in the middle flow of the flow path, there is a problem that bubbles are more likely to remain downstream of each air hole or between the air holes.

Since bubbles have the property of easily moving in the liquid, where bubbles remain in the flow path while the sample is filled in the flow path, the bubbles may move in the sample, which may be accompanied by the relative movement of the position of the sample filled in the flow path. In particular, when bubbles remain near the air hole, the bubbles are likely to be discharged from the air hole, and in addition, since the bubbles move vigorously, the amount of movement of the sample increases. When such a phenomenon occurs, the measuring object component that has reacted with a reagent present in the measurement region may move out of the measurement region (toward downstream of the measurement region). Further, when the movement of bubbles occurs during the measurement of the object component, the measuring object component, which is the measuring object in the measurement region, may change. As a result, there is a possibility that the measured values of the measuring object in the sample may vary. Therefore, there is a need for a shape such that bubbles do not remain in the flow path of the biosensor.

An object of the practical example of the present invention is to provide a biosensor in which bubbles are unlikely to remain in a flow path.

One aspect of the practical example of the present invention is a biosensor for measuring a measuring object component contained in a sample. This biosensor includes a substrate which has an upper surface on which a measurement region is formed, a spacer which has a cutout portion obtained by cutting out a part of the spacer and which is stacked on the upper surface so that the measurement region is located inside the cutout portion, and a cover which has an air hole obtained by cutting out a part of the cover and which is stacked on the spacer while covering the cutout portion, wherein the substrate, spacer, and cover form a flow path for the sample, the flow path being formed to have a supply port for supplying the sample and communicate with the air hole. The side wall of the flow path is constituted by the cutout portion, and a downstream edge portion of the cutout portion forming a downstream end of the flow path and an exposed portion, which is a part of the spacer continuous from the downstream edge portion, protrude into the air hole in a top view of the air hole.

According to the practical example of the present invention, it has become possible to provide a suitable biosensor capable of preventing bubbles from remaining in the flow path.

### BRIEF DESCRIPTION OF THE DRAWINGS

FIG. 1 is an exploded perspective view showing a configuration example of a biosensor according to an embodiment;
FIG. 2 is a plan view showing a configuration example of a part of the biosensor according to the embodiment;
FIG. 3 is a cross-sectional view when the part of the biosensor according to the embodiment shown in FIG. 2 is cut along the A-A line;
FIG. 4 is a diagram showing a pattern of the shape and position of the air hole evaluated in Test 1;
FIG. 5 is a detailed explanatory view of the shape of the air hole in the case of a circle and in the case of an oval; and
FIG. 6 is a graph showing the time course of a response signal related to the hematocrit measurement when there are no remaining bubbles evaluated in Test 3 and when the sample is moved by the bubbles remaining in the flow path.

### DESCRIPTION OF THE EMBODIMENTS

In the embodiment, a biosensor for measuring a measuring object component contained in a sample will be described. This biosensor features the following. Thus, the biosensor comprises a substrate having an upper surface on which a measurement region is formed, a spacer which has a cutout portion obtained by cutting out a part thereof and which is stacked on the upper surface so that the measurement region is located inside the cutout portion, and a cover which has an air hole obtained by cutting out a part thereof and which is stacked on the spacer while covering the cutout portion. The substrate, spacer, and cover form a flow path for a sample that has a supply port for supplying the sample and communicates with the air hole, the side wall of the flow path is constituted by the cutout portion, and a downstream edge portion of the cutout portion forming a downstream end of the flow path and an exposed portion which is a part of the spacer continuous from the downstream edge portion protrude into the air hole in the top view of the air hole.

According to the biosensor having the above-mentioned configuration, since the downstream edge portion and the exposed portion protrude into the air hole in the top view of the air hole, the air in the flow path can be suitably released from the air hole. This makes it possible to prevent the bubbles from remaining in the flow path. Further, in the biosensor, the dimension of the air hole in the sample supply direction on the substrate surface may be larger than the dimension in the direction orthogonal to the supply direction. In other words, in the biosensor, the dimension of the air hole in the sample supply direction may be larger than the dimension in the direction perpendicular to the supply direction in the horizontal direction (the direction orthogonal to the supply direction in the plane including the supply direction). For example, the air hole is formed into a shape inclusive of a rectangle or an oval that is long in the sample supply direction. As a result, compared to the case where the shape of the air hole is circular or square, even if the position of the air hole is slightly shifted in the sample supply direction due to an error during manufacturing (difference between lots), a state of overlapping with the edge of the cutout portion can be realized. Therefore, it becomes possible to easily form a suitable biosensor in which bubbles can be prevented from remaining.

The sample is, for example, a biological sample. Biological samples include, for example, liquid samples such as blood, interstitial fluid, urine, and the like. The measuring object components include glucose (blood glucose), lactate (lactic acid), or the ratio (hematocrit value) of blood cells (hematocrit).

The biosensor can adopt the following configuration. That is, the surface of the exposed portion is surface-modified, and the contact angle of the surface of the exposed portion is larger than the contact angle of the spacer portion that is not surface-modified. As an example, the air hole is formed by irradiating the cover stacked on the spacer with a laser beam, and the contact of the spacer portion deformed by the incident laser light penetrating the cover when the air hole is formed is larger than the contact angle of the spacer portion that has not been deformed by the laser beam. The portion onto which the laser beam is incident is deformed by the heat of the laser beam to form highly hydrophobic irregularities. As a result, the contact angle of the portion of the spacer onto which the laser beam is incident is larger than the contact angle of the portion onto which the laser beam is not incident, so that the sample in the flow path stops at the downstream edge, which is the edge in the sample supply direction of the cutout portion forming the downstream end of the flow path, and is prevented from overflowing from the air hole. As another example, a biosensor may be used that is manufactured by using a spacer that is surface-modified to increase the contact angle of the area including the surface of the exposed portion before stacking on the substrate.

Hereinafter, the biosensor in the embodiment will be described with reference to the drawings. The configuration of the embodiment described hereinbelow is exemplary, and the present invention is not limited to the configuration of the embodiment.

In the following embodiment, a biosensor in which blood (whole blood) can be used as a sample to measure the glucose value in blood as a measuring object component, the hematocrit value in the blood can be also measured, and the glucose value can be corrected by the hematocrit value will be explained as an example of a biosensor as an analytical tool,

### Biosensor Configuration

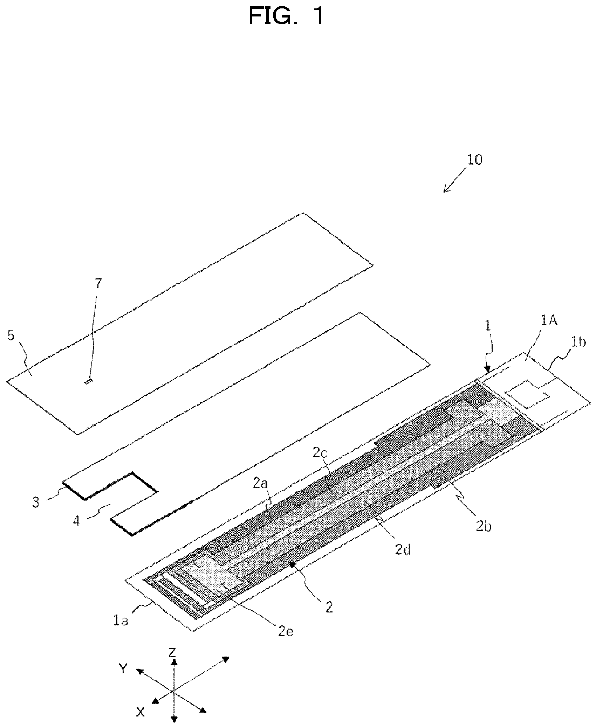

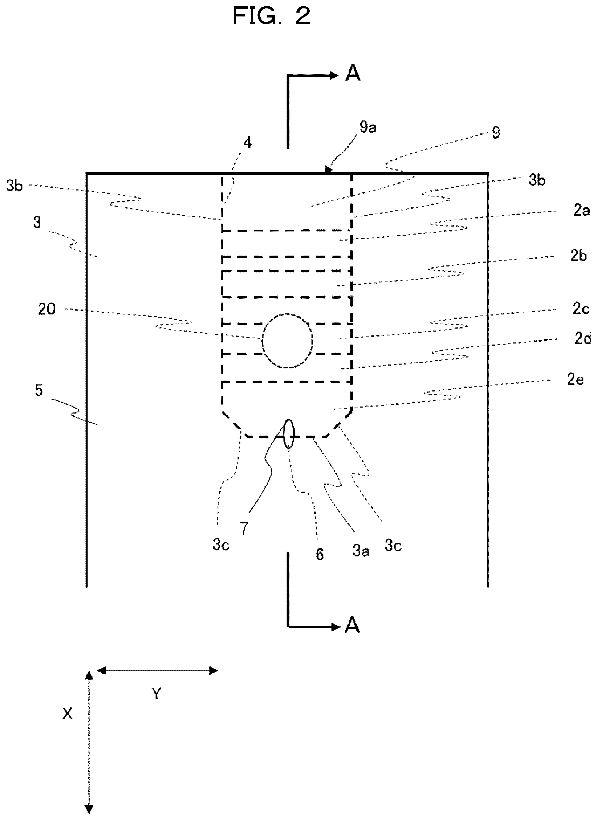

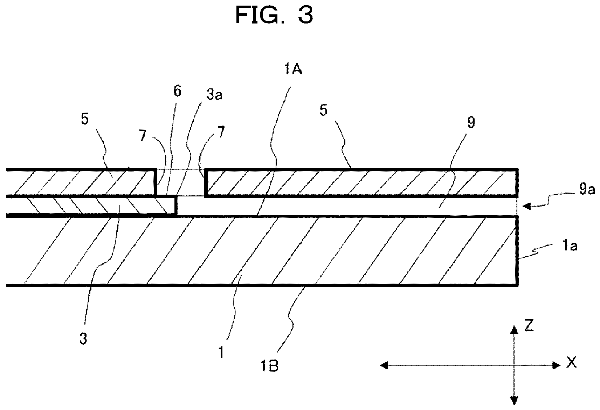

FIGS. 1, 2 and 3 show a configuration example of the biosensor according to the embodiment. FIG. 1 shows an exploded perspective view of the biosensor. FIG. 2 is a plan view showing a part of the biosensor, and shows the configuration of the flow path. FIG. 3 shows a cross section of the biosensor shown in FIG. 2 when it is cut along the A-A line. In FIG. 3, an electrode layer 2 and a reagent 20 are not shown.

In FIG. 1, the biosensor 10 has a length direction (X direction), a width direction (Y direction), and a height direction (Z direction). It can also be said that the length direction (X direction) is the sample supply direction, and the width direction (Y direction) is the direction perpendicular to the sample supply direction in the horizontal direction (that is, the direction orthogonal to the sample supply direction in the horizontal plane including the sample supply direction) and the height direction (Z direction) is the stacking direction. The biosensor 10 is formed by stacking an insulating substrate 1 having the electrode layer 2, a spacer 3, and a cover 5 in the Z direction and adhesively bonding.

The substrate 1 is formed as a flat plate of a planar rectangular shape having a length direction and a width direction and having one end 1a and the other end 1b in the length direction. The substrate 1 has an upper surface 1A on which the electrode layer 2 is formed and a lower surface 1B on the side opposite that of the upper surface 1A.

The substrate 1 is formed by using, for example, a synthetic resin (plastic). Various resins, for example, such as polyetherimides (PEI), polyethylene terephthalate (PET), polybutylene terephthalate (PBT), polyethylene (PE), polystyrene (PS), polymethacrylates (PMMA), polypropylene (PP), polyimide resins, acrylic resins, epoxy resins and glass epoxy can be adopted as the synthetic resin. However, an insulating material other than the synthetic resin can also be adopted for the substrate 1. Insulating materials include, in addition to synthetic resins, paper, glass, ceramics, biodegradable materials, and the like. In this embodiment, the substrate 1 is formed of polyethylene terephthalate (PET).

The biosensor of the present invention has a measurement region formed as a region for measuring the measuring object component contained in the sample, and in the biosensor of the present embodiment, the measurement region on which the electrode layer 2 is formed is provided on the upper surface 1A of the substrate 1. In the present embodiment, the electrode layer 2 includes electrodes 2a to 2e. FIG. 2 shows a part of the biosensor 10 and shows a state of a portion on the one end 1a side of the substrate 1 on which the spacer 3 and the cover 5 are stacked. The electrode 2a is a working electrode for measuring hematocrit. The electrode 2b is a counter electrode for measuring hematocrit. The electrode 2c is a working electrode for measuring glucose. The electrode 2d is a counter electrode for measuring glucose. The electrode 2e is a sample detection electrode. In the biosensor of the present invention, the region where the electrodes 2a to 2e exposed in the flow path are formed is the measurement region. However, the number and types of electrodes are not limited to the above examples.

The electrode layer 2 is formed by using, for example, a metal material such as gold (Au), platinum (Pt), silver (Ag), palladium, ruthenium, and nickel, an alloy thereof, or a carbon material such as carbon. In the present embodiment, a nickel vanadium alloy as a metal material is deposited on the upper surface 1A of the substrate 1 by physical vapor deposition (PVD, for example, sputtering) or chemical vapor deposition (CVD) to form a metal layer having a desired thickness, and electrodes 2a to 2e are formed by drawing a predetermined electrode pattern with a laser beam. However, the present invention is not limited to the above example, and the electrode material may be formed, for example, by printing on the substrate 1 by screen printing.

The spacer 3 has a U-like shape having a cutout portion 4, and is used for forming a flow path 9 for a sample, which will be described hereinbelow. The cover 5 is superposed on the spacer 3. For this purpose, the cover 5 has a rectangular shape that matches the planar shape of the spacer 3. The spacer 3 and the cover 5 may or may not be integrally formed. In the present embodiment, the spacer 3 is formed of an acrylic resin (PMMA) having a contact angle (hydrophobicity) lower than that of the cover 5, and the cover 5 is formed of a hydrophilic film.

By stacking the substrate 1 on which the electrode layer 2 is formed, the spacer 3, and the cover 5, a space having a supply port 9a formed by the cutout portion 4 of the spacer 3 is formed on one end 1a side (one end side in the length direction) of the substrate 1. That is, the space surrounded by the bottom surface formed by the upper surface 1A exposed by the cutout portion 4 (not covered by the spacer 3), the side walls due to the thickness of the spacer 3 (the side walls formed by the inner surface of the cutout portion 4), and the ceiling surface (upper wall) due to the lower surface of the cover 5 is formed. This space is used as the flow path 9 for the sample. The flow path 9 has 3.7 mm in the X direction and 1.8 mm in the Y direction. The supply port 9a is used as an opening for supplying the sample to the flow path, in other words, as a sample supply port for supplying the sample to the biosensor. The supply port 9a is not limited to being formed on the side surface in the length direction of the biosensor as illustrated in FIG. 1. For example, a cutout that is an opening for the supply port 9a may be formed on the side surface in the width direction of the biosensor, the substrate 1, or the cover 5, thereby obtaining the shape that enables the supply of the sample into the flow path 9 from the height direction.

As shown in FIG. 2, the edge portion of the cutout portion 4 forming the side walls of the flow path 9 is composed of a widthwise edge portion 3b extending in the X direction which is the length direction, a downstream edge portion 3a that extends in the Y direction which is the width direction and constitutes the downstream end of the flow path, and a connecting edge portion 3c connecting the widthwise edge portion 3b and the downstream edge portion 3a. The downstream edge portion 3a, the widthwise edge portion 3b, and the connecting edge portion 3c partition the portion of the electrode layer 2 exposed to the flow path 9 and the portion of the electrode layer 2 covered by the spacer 3 to constitute the side walls of the flow path 9. The connecting edge portion 3c may be omitted, and the widthwise edge portion 3b and the downstream edge portion 3a may be directly connected to each other. It can also be said that the connecting edge portion 3c is a part of the downstream end of the flow path.

The cover 5 is formed with an air hole 7 that penetrates the cover 5 and leads to the flow path 9. The side wall of the air hole 7 is formed along the Z direction, and the opening on the upper side and the opening on the lower side of the air hole 7 have substantially the same area. When viewed from the direction opposite to the direction in which the substrate 1, the spacer 3, and the cover 5 are stacked (stacking direction), which is the height direction (Z direction) of the biosensor 10, that is, in the plan view of the biosensor 10, the downstream edge portion 3a of the cutout portion 4 forming the downstream end of the flow path 9, and a part of the spacer 3 continuous from the downstream edge portion 3a protrude into the air hole 7. In other words, when the air hole 7 or the cover 5 is viewed from directly above (when the air hole 7 or the cover 5 is viewed from the top (planar view)), the air hole 7 exposes the downstream edge portion 3a of the cutout portion 4 forming the downstream end of the flow path 9, and the upper surface (referred to as an exposed portion 6) of the spacer 3 continuous from the downstream edge portion 3a, and these are exposed to the outside by the air hole 7. It can be said that a part of the opening on the lower side (the part communicating with the flow path 9) of the air hole 7 is closed by a part of the spacer 3 corresponding to the exposed portion 6. Further, it can be said that the air hole 7 overlaps (intersects) with the downstream edge portion 3a of the cutout portion 4 when viewed in the stacking direction.

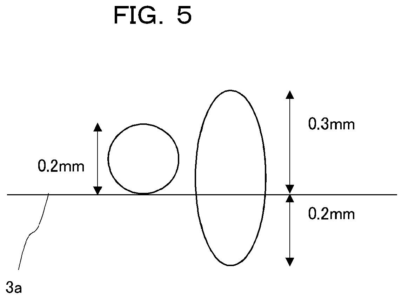

As an example, the air hole 7 is formed to have a shape such that the dimension in the X direction, which is the length direction, is larger than the dimension in the Y direction, which is the width direction. For example, as shown in FIG. 5, the air hole is formed in an oval shape that is 0.5 mm in the X direction and 0.2 mm in the Y direction and has an area of about 0.09 mm². In FIG. 5, the proportion of the flow path communication portion, where the spacer 3 is not covered by the air hole 7 and the upper surface of the flow path 9 is exposed when viewed in the stacking direction, and the protruding portion, where a part of the spacer 3 continuous from the downstream edge portion 3a protrudes into the air hole 7 and the exposed portion 6 is exposed when viewed in the stacking direction, and the area of each portion, are not particularly limited, and conditions are set such that the air filled in the flow path 9 when the sample is introduced into the flow path 9 is easily discharged from the air hole 7 and bubbles are unlikely to remain in the flow path 9. In the biosensor of the present embodiment, the position of overlapping with the downstream edge portion 3a is set so that the ratio of the flow path communication portion of the oval air hole 7 shown in FIG. 5 and the protruding portion is 3 : 2. However, the shape of the air hole 7 is not limited to the oval shape shown in FIG. 5 and may be any shape, such as a circular shape, a polygon such as a triangle or a rectangle, etc., provided that the downstream edge portion of the cutout portion and the exposed portion continuous from the downstream edge portion can protrude into the air hole 7 in the top view of the air hole. Further, not only one, but a plurality of the air holes 7 may be formed. Further, the air hole 7 is formed so as to be located at the center of the flow path 9 in the direction perpendicular to the sample supply direction (that is, the Y direction) in the horizontal plane including the sample supply direction (that is, the X direction).

As shown in FIG. 2, a part of the electrode layer 2 (electrodes 2a to 2e) is exposed in the flow path 9. The reagent 20 is provided (immobilized) by placing on the electrode 2c and electrode 2d (working electrode and counter electrode for measuring glucose). Meanwhile, no reagent is provided on the electrode pair (electrodes 2a and 2b) for measuring hematocrit. However, the reagent may be provided thereon. The electrode 2e is used to detect that the sample has been introduced into the flow path 9.

The reagent 20 includes an enzyme. The reagent 20 may further include a mediator. The enzyme is selected, as appropriate, according to the type of sample and the measuring object component. When the measuring object component is glucose in blood or interstitial fluid, glucose oxidase (GOD) or glucose dehydrogenase (GDH) is adopted. The mediator is, for example, a ferricyanide, p-benzoquinone, a p-benzoquinone derivative, phenazine methosulfate, methylene blue, ferrocene, a ferrocene derivative, a ruthenium complex, and the like. Of these, the ruthenium complex is more preferred.

Further, the end portion of the electrode layer 2 (electrodes 2a to 2e) located on the other end 1b side of the substrate 1 is exposed without being covered by the spacer 3 or the cover 5. When the biosensor 10 is attached to a measuring device for measuring the glucose concentration and the hematocrit value, the ends of the electrodes 2a to 2e are electrically connected to a circuit in the measuring device.

The biosensor 10 is used in a state where the biosensor 10 is attached to the measuring device. When starting the measurement, first, the sample (blood) is brought into contact with the supply port 9a in this attached state. Then, the sample is drawn into the flow path 9 by the capillary force of the flow path 9, flows to the other end 1b side of the substrate 1, and fills the inside of the flow path 9. At this time, the air filled in the flow path 9 is pushed out by the sample and discharged from the air hole 7. The sample that fills the flow path 9 comes into contact with the electrodes 2a to 2e and also comes into contact with the reagent 20 placed on the electrodes 2c and 2d to dissolve the reagent 20. In the measuring device, when the sample is introduced into the flow path 9, the electrode 2e and any of the other electrodes (2a to 2d) are electrically connected which is detected by an electric signal, and the measurement is started.

For example, in the measuring device, first, a DC voltage (first voltage) is applied between the electrode 2a and the electrode 2b, which are a pair of electrodes for measuring hematocrit, and a response signal to the first voltage application is measured as a response current value. The measuring device calculates the hematocrit value from the response current value of the first voltage application. Next, in the measuring device, a DC voltage (second voltage) is applied between the electrodes 2c and 2d, which are a pair of electrodes for measuring glucose, and a response signal to the second voltage application is measured as a response current value. The measuring device converts the response current value of the second voltage application into the glucose concentration. Further, the converted value of the glucose concentration is corrected by the hematocrit value. The glucose concentration may also be corrected by using the response current value of the first voltage application instead of the hematocrit value.

Such a method for measuring glucose concentration is exemplary, and in the biosensor of the present invention, a measurement method for measuring a measuring object component contained in the sample such as glucose concentration in blood, the shape of the measurement area for carrying out the measurement method, the type of the reagent, and the installation method can take various forms. For example, the biosensor is not limited to the electrode method using electrodes, and a colorimetric method for detecting a reaction between a measuring object component and a reagent may be used as the measurement method. In the case of the colorimetric method, the region in the flow path where the reagent is placed becomes the measurement region, and the measurement is performed by detecting the change in color tone caused by the reaction. In a biosensor using the colorimetric method as a measurement method, the reagent may be placed on the substrate 1 and the electrode layer may not be formed. The biosensor of the present invention is also useful when the colorimetric method is used because the air remaining in the measurement region affects the detected value of the color tone change.

### Method for Manufacturing Biosensor

The biosensor 10 can be manufactured, for example, by the following manufacturing method. First, a resin sheet (substate sheet) having a metal film formed on one side (upper surface) is prepared. Next, by irradiating the metal layer of the substrate sheet with laser light using a laser light irradiation device and removing the metal film at that portion, an electrode pattern of the electrode layer 2 consisting of m rows × n columns (m and n are integers of 1 or more) is drawn. Then, the reagent 20 is placed and formed on the electrode 2c and the electrode 2d (working electrode and counter electrode for measuring glucose).

Next, a material serving as a spacer 3 having a plurality of cutout portions obtained by cutting out parts thereof is stacked on the upper surface of the substrate sheet so that each measurement region is located inside the corresponding cutout portion 4, and then a material serving as the cover 5 is stacked on the spacer 3 so as to cover the cutout portions 4. As a result, the flow path 9 having side walls formed by the cutout portion 4 and having the supply port 9a to which the sample is to be supplied is formed in each biosensor. At this time, where PMMA is used as the material of the spacer 3, the substrate sheet and the material of the cover 5 are adhered to each other due to the adhesiveness of PMMA. At this time, a spacer 3 and a cover 5 stacked in advance may be adhered to the upper surface of the substrate sheet.

Next, for each biosensor on the substrate sheet, a laser beam is radiated using a laser irradiation device to form the air hole 7 penetrating the cover 5. At this time, the air hole 7 is formed by penetrating and cutting a part of the cover 5 with the laser beam so as to communicate with the flow path 9 and so that the downstream edge portion 3a of the cutout portion 4 forming the downstream end of the flow path 9 and the exposed portion 6, which is a part of the spacer 3 continuous from the downstream edge portion 3a, protrude when viewed in the stacking direction. Further, by moving the laser irradiation device (laser beam) relative to each biosensor along the length direction of the biosensor, the air hole 7 is formed in which the dimension in the length direction (X direction) is larger than the dimension in the width direction (Y direction). As a result, the air hole 7 is formed in which the dimension in the length direction is larger than the dimension in the width direction and which overlaps with the edge of the cutout portion extending in the width direction in a plan view in which the spacer 3 and the cover 5 are superposed on the electrode layer.

The irradiation with the laser beam is preferably performed so that, the laser beam penetrates the material of the cover 5 and is incident on the spacer 3 located thereunder, that is, the surface of the exposed portion 6, whereby the surface portion of the exposed portion 6 onto which the laser beam is incident is deformed by the heat of the laser beam and irregularities that improve the hydrophobicity are formed. As a result of the improvement of hydrophobicity due to deformation, the contact angle of the portion deformed by the surface modification by the laser beam becomes larger than the contact angle of the portion not deformed by the laser beam. The contact angle is measured by bringing water, which is the sample, into contact with the spacer 3.

Explained hereinbelow is the principle according to which the contact angle of the portion deformed by surface modification by the laser beam becomes larger than the contact angle of the portion not deformed by the laser beam, for example, in the case where the spacer 3 is formed by PMMA used as a pressure-sensitive adhesive. That is, the spacer 3 is melted by the heat of the laser beam beyond the glass transition temperature and is supercooled. As a result, the arrangement of molecules in the portion of the spacer 3 deformed by the laser beam becomes non-uniform (amorphous), and physical properties (generally, the strength can be mentioned) change. The portion deformed by the laser beam is accordingly cured to have a structure with increased hydrophobicity, so that the contact angle increases. It can be said that hydrophobic irregularities are formed (hydrophobic wall is formed). The glass transition temperature of PMMA is 90 degrees (generally 50 degrees to 150 degrees). The deformation of the material of the spacer 3 by the laser beam is not limited to the case where the material of the spacer 3 is PMMA, and any material other than PMMA that can form irregularities with increased hydrophobicity as a result of deformation may be used.

After that, the biosensors 10 having the configuration described with reference to FIGS. 1 to 3 are formed by cutting out individual pieces of m × n biosensors by shearing the substrate sheet. The supply port 9a of the flow path 9 may be formed when the material of the spacer 3 is stacked on the upper surface of the substrate sheet or when the individual pieces are cut out.

The air hole 7 is not limited to the method of forming by a laser beam penetrating the cover 5, and a physical means such as punching by press working may be used. Further, the method of forming the air hole 7 after stacking the spacer 3 and the cover 5 on the upper surface of the substrate sheet is not limiting, and it is also possible to form the air hole 7 in the cover 5 by any means in advance and stack the cover 5 by adjusting the position so that the air hole 7 communicates with the flow path 9, and the downstream edge portion 3a and the exposed portion 6 protrude into the air hole 7 when viewed in the stacking direction. In that case, the portion of the spacer 3 exposed from the air hole 7 may be subjected to a hydrophobic surface modification treatment in advance.

### Examples

### Test 1

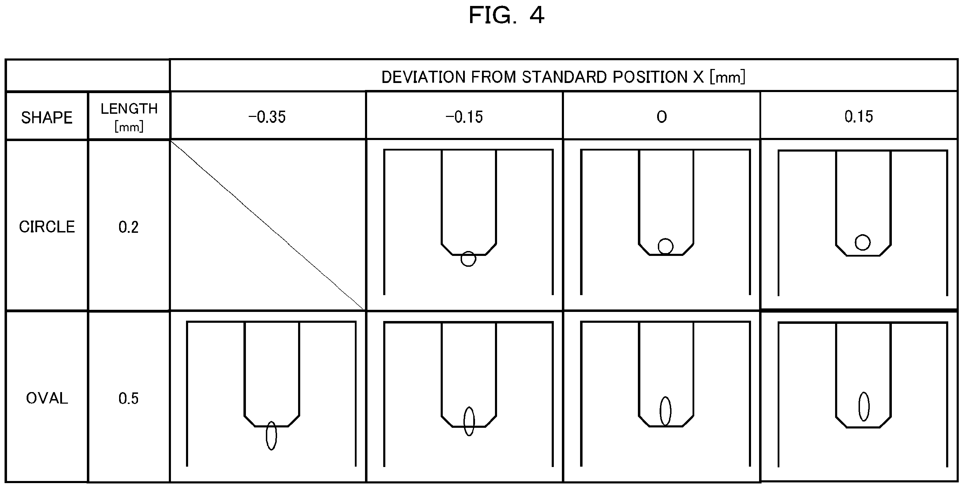

The following test (Test 1) was conducted to confirm the effect of the biosensor 10. A plurality of biosensors having different shapes and positions of air holes was prepared as the biosensor 10 for the test. FIG. 4 shows the patterns of shapes and positions of the air hole, and FIG. 5 is an enlarged view showing the shapes of the air hole at the standard position described hereinbelow and the position relative to the downstream edge portion 3a.

As shown in FIG. 5, as the shapes of the air hole, a circular shape (on the left side in FIG. 5) as a comparative example and an oval shape (on the right side in FIG. 5) as an example were prepared. The circular air hole was formed by irradiating with the laser irradiation device that was not moved with respect to the biosensor, and the oval air hole was formed by moving the laser irradiation device by 0.3 mm relative to each biosensor along the length direction (X direction) of the biosensor. The circular shape has a diameter of 0.2 mm, and the oval shape has a dimension in the length direction (X direction) larger than in the width direction (Y direction), a major axis of 0.5 mm, and a minor axis of 0.2 mm. Since the air holes are formed by the laser irradiation device and the exposed portion is also irradiated with the laser, the exposed portion is surface-modified to be hydrophobic.

For the air hole, the state of contact with the downstream edge portion 3a at the other end 1b side of the substrate 1, which is opposite to the one end 1a (supply port 9a) side of the substrate 1 in the length direction (X direction), is defined as the standard position.

As shown in FIG. 4, the deviation from the standard position to the other end 1b side in the X direction was taken as a negative deviation, and the deviation to the one end 1a side was taken as a positive deviation. It was assumed that there was no deviation in the Y direction. Table 1 below shows the relationship between the magnitude of the deviation of the air holes in the negative or positive direction and the state of bubbles remaining in the flow path 9.

**[Table 1]**

| SHAPE | LENGTH [mm] | DEVIATION FROM STANDARD POSITION X [mm] | | | | | | | | | | |
|---|---|---|---|---|---|---|---|---|---|---|---|---|
| | | -0.35 | -0.3 | -0.25 | -0.2 | -0.15 | -0.1 | -0,05 | 0 | 0.05 | 0.10 | 0.15 |
| CIR-CLE | 0.2 | - | | - | - | NG | NG | ○ | △ | △ | △ | × |
| OVAL | 0.5 | ○ | ○ | ○ | ○ | ○ | ○ | ○ | △ | △ | × | × |

| | | | | | | | | | | | | |
|---|---|---|---|---|---|---|---|---|---|---|---|---|
| ○ : Bubbles do not remain △ : The proportion of remaining bubbles is low × : The proportion of remaining bubbles is high NG: Processing is not good -: No data | | | | | | | | | | | | |

In the case of a circular air hole, the following results were obtained. Bubbles remained in a low proportion at the standard position in Test 1, and when there was a negative deviation therefrom, no bubbles remained at -0.05 mm, and with a deviation of more than -0.1 mm, the air hole did not function (was not formed) and the processing was not good. When the deviation was positive, the proportion of remaining bubbles was low in the range of 0.05 mm to 0.10 mm and high at 0.15 mm. Meanwhile, in the case of an oval air hole, the result was that bubbles remained in a low proportion at the standard position, and in the range of -0.05 mm to -0.35 mm evaluated in the case of negative deviation from the standard position, no bubbles remained for any of these positions. In the case of a positive deviation, the result was that the proportion of remaining bubbles was low at 0.05 mm and high at 0.10 mm to 0.15 mm. Thus, it was found that bubbles can be prevented from remaining inside the flow path 9 in a state in which the air hole overlaps (intersects) with the downstream edge portion 3a under the condition that the air hole functions, that is, in a state in which the downstream edge portion 3a of the cutout portion 4 forming the downstream end of the flow path 9 and a part of the spacer 3 continuous from the downstream edge portion 3a protrude when viewed in the stacking direction. The air hole functions when the air filled in the flow path is pushed out to the sample and discharged. "Under the condition that the air hole functions" indicates that the area where the flow path is exposed from the air hole, in other words, the area where the upper surface 1A of the substrate 1 is exposed from the air hole in a plan view is sufficiently large to push out the air filled in the flow path from the air hole. In addition, it is understood that the oval air hole has a wider range of allowed positional deviation in the X direction than the circular air hole. That is, by forming the oval air hole, the processing accuracy at the time of forming the air hole is relaxed.

### Test 2

A contact angle formed by forming the air hole 7 by irradiating with the laser beam penetrating the cover 5 and dropping water on the portion of the spacer 3 deformed by surface modification by such laser beam irradiation, and a contact angle formed by dropping water on the portion of the spacer 3 that was not deformed (was not processed) by the laser beam were measured. In the case of the unprocessed portion, the contact angle was 85.96°, and in the case of the spacer deformed by laser irradiation, the contact angle was 92.7°. Therefore, it is understood that the contact angle is increased due to the deformation caused by the laser irradiation, the sample moving toward the air hole 7 is repelled, and the sample can be prevented from overflowing from the air hole 7. In the case of not only forming the exposed portion 6 of the spacer 3 that protrudes into the air hole 7, but also making the exposed portion 6 hydrophobic, the exposed portion 6 can prevent the sample from overflowing from the air hole 7.

### Test 3

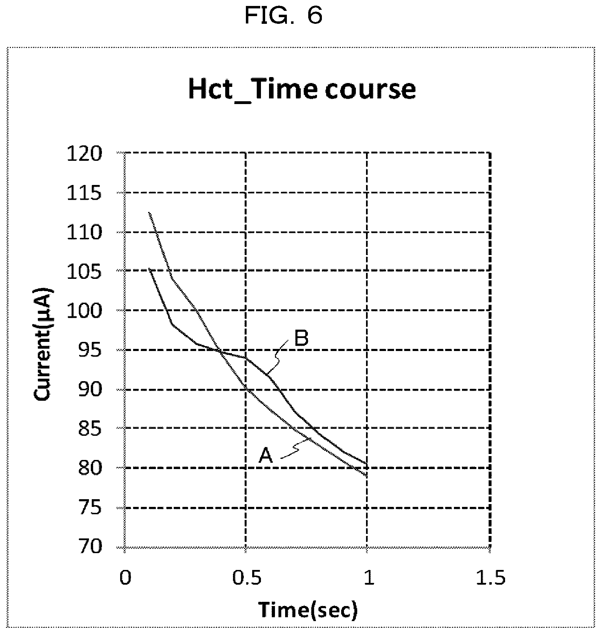

FIG. 6 is a graph showing the temporal change of the response current value with respect to the application of the first voltage at the time of hematocrit measurement. Graph A in FIG. 6 shows the measurement result when there are no bubbles remaining in the flow path 9 (normal case), and Graph B shows the measurement result when the bubble remaining in the flow path moved in the flow path 9 in the vicinity of 0.25 sec.

As shown in FIG. 6, in the normal case, as shown in Graph A, the response current value smoothly and monotonically decreased according to a certain functional formula, whereas where the bubbles remaining in the flow path moved in the flow path 9, the graph of the response current value assumed an irregular shape as shown in Graph B rather than following a monotonically decreasing function. In the case of FIG. 6, the response current value decreased monotonically up to around 0.25 sec, but from the vicinity of 0.25 sec when the movement of bubbles started to the vicinity of 0.5 sec, the amount of change in the response current value was small, and then the response current value decreased monotonically again from the vicinity of 0.6 sec. Such a phenomenon occurs because the sample (blood) present between the electrodes 2a and 2b for measuring hematocrit moves during the hematocrit measurement due to the movement of the bubbles. That is, the sample in the flow path moves as a whole to the downstream side due to the movement of bubbles, and since the sample is a liquid, fluctuations (waves) are generated in the sample. This is due to the occurrence of the effect of changes in the sample that was present on the electrode, which is the measurement area, before and after the movement of bubbles, and the electrical effect (effect on applied voltage and effect of response current value acquisition) due to fluctuation (wave) of the sample during and immediately after the movement of bubbles. Since the response current value corresponding the hematocrit or the application of the first voltage is used for the correction of glucose concentration, the remaining bubbles such as shown in Graph B affect the measurement accuracy of the hematocrit and the glucose concentration. Such a tendency can occur similarly when measuring glucose by using a pair of glucose electrodes. In the biosensor 10 according to the embodiment, since bubbles can be prevented from remaining in the flow path, the sample does not move during the measurement, and there is low probability of affecting the measurement accuracy of the measuring object component such as the hematocrit value and the glucose concentration. The configurations described in the embodiments can be combined as appropriate.

## Claims

1. A biosensor for measuring a measuring object component contained in a sample, the biosensor comprising:
a substrate which has an upper surface on which a measurement region is formed;
a spacer which has a cutout portion obtained by cutting out a part of the space and which is stacked on the upper surface so that the measurement region is located inside the cutout portion; and
a cover which has an air hole obtained by cutting out a part of the cover and which is stacked on the spacer while covering the cutout portion, wherein
the substrate, the spacer, and the cover form a flow path for the sample, the flow path being formed to have a supply port for supplying the sample and communicate with the air hole, and the side wall of the flow path is constituted by the cutout portion, and
a downstream edge portion of the cutout portion forming a downstream end of the flow path and an exposed portion, which is a part of the spacer continuous from the downstream edge portion protrude, into the air hole in a top view of the air hole.

2. The biosensor according to claim 1, wherein a dimension of the air hole in a supply direction of the sample is larger than the dimension in the direction orthogonal to the supply direction on the substrate surface.

3. The biosensor according to claim 2, wherein the air hole has an oval shape in which the dimension in the sample supply direction is larger than the dimension in the direction orthogonal to the supply direction.

4. The biosensor according to claim 2, wherein the air hole is formed so as to be located at the center of the flow path in a direction perpendicular to the supply direction in a horizontal plane including the sample supply direction.

5. The biosensor according to any one of claims 1 to 4, wherein
a surface of the exposed portion has been surface-modified, and
a contact angle of the surface of the exposed portion is larger than a contact angle of the spacer portion that has not been surface-modified.

6. The biosensor according to any one of claims 1 to 5, wherein the air hole is formed by irradiation with a laser beam.
